# EUROPEAN PATENT APPLICATION

(11) **EP 4 174 162 A1**
(43) Date of publication of application: **03.05.2023**
(21) Application number: 21834157.6
(22) Date of filing: 28.06.2021
(51) Int. Cl.: C12M 1/00, C12Q 1/68, G01N 35/00

(54) **INTERNAL FIXING DEVICE FOR APPARATUS IN TRANSPORT MODE, APPARATUS COMPRISING INTERNAL FIXING DEVICE, AND METHOD FOR REDUCING DAMAGE TO APPARATUS DURING TRANSPORT**

(30) Priority: 30.06.2020 CN 202021253349 U
(71) Applicant: Leadway (HK) Limited, Hong Kong (CN)
(72) Inventor: MAO, Haiming, Hangzhou, Zhejiang 310030 (CN); LI, Zhiguang, Hangzhou, Zhejiang 310030 (CN); WAGN, Tao, Hangzhou, Zhejiang 310030 (CN); YU, Chun, Hangzhou, Zhejiang 310030 (CN); YU, Fangmin, Hangzhou, Zhejiang 310030 (CN); RONG, Fanwen, Hangzhou, Zhejiang 310030 (CN); JIANG, Guochun, Hangzhou, Zhejiang 310030 (CN)
(74) Representative: Schiweck Weinzierl Koch Patentanwälte Partnerschaft mbB
(86) International application number: PCT/CN2021/102862
(87) International publication number: WO 2022/001990

(57) **Abstract**

The present invention relates to an internal fixing device for an apparatus in a transport mode, an apparatus comprising said device, and a method for reducing the risk of damage to the apparatus during transport. The internal fixing device comprises a limiting device, eccentric roller transmission assemblies, a motor assembly, a motion guide assembly, etc. The two ends of a motor output shaft are fixedly disposed on eccentric roller transmission assemblies on two sides of the apparatus. By triggering a microswitch on the limiting device, eccentric rollers and rocking handles form self locking in a transport mode to stop motion. The present invention can effectively reduce the risk of damage to the apparatus during transport.

## Description

### Field of the Invention

The present invention relates to the field of internal fixing of an apparatus, in particular to an internal fixing device for an apparatus during transport and a method for reducing damage to the apparatus during transport, and especially an internal fixing device for a nucleic acid analyzer, and a method for reducing damage to the nucleic acid analyzer during transport.

### Background of the Invention

The PCR technology has a principle similar to the natural replication process of DNA, its specificity depends on oligonucleotide primers complementary at the two ends of a target sequence, and it consists of three basic reaction steps: denaturation, annealing and extension. The RT-PCR technology is developed by combining RNA reverse transcription with PCR. Based on continuous development of PCR and RT-PCR detection techniques, multiple nucleic acid detection methods such as fluorescent PCR, DNA sequencing and RNA sequencing emerge, which have characteristics of high sensitivity and high specificity and the like and have been widely used in human and animal disease diagnosis (e.g., prenatal diagnosis, newborn screening and genetic metabolic disease detection), forensic detection, consanguinity analysis, seed purity identification, molecular marker-assisted breeding, gene mapping, genetically modified organism detection, etc. Whatever PCR or RT-PCR is performed in a PCR analyzer. As an apparatus for amplification of specific DNA or RNA, a PCR instrument mainly includes a sample compartment, a hot cover module and a control module. A PCR instrument for fluorescent PCR detection (also referred to as a fluorescent PCR instrument) further includes an optical detection module, and can use an excitation light source to irradiate a reaction tube in a PCR/RT-PCR amplification reaction to excite fluorescent dyes or fluorophore-containing probes (such as TaqMan or MGB probes, two-hybrid probes, or molecular beacon probes, etc.) to emit fluorescence, detect the resulting fluorescent signals, and monitor the entire PCR/RT-PCR reaction process in real time using accumulation of the fluorescence signals to qualitatively or quantitatively measure the DNA or RNA that may be present in a clinical sample.

When the PCR instrument is in the working mode, under the driving of a moving mechanism, a test tube stand or test tube holder in a thermal cycling module is connected with the hot cover module, and the PCR reaction reagent (which includes DNA polymerase, at least one pair of amplification primers, magnesium ions, dNTPs, etc., preferably, further includes at least one fluorophore-containing probe, and should further include reverse transcriptase if RNA is amplified) in the test tube and the extracted sample nucleic acid are subjected to multiple rounds of nucleic acid amplification reactions. For example, Chinese patent CN2218181Y discloses that a test tube stand is connected to a transmission device mounted on a test tube holder that drives the test tube stand to move up and down; the test tube holder is mounted on the horizontal guide rail of a rack and connected to a drive device that drives the test tube holder to move horizontally; and the transmission device is a crank slider mechanism or eccentric wheel mechanism and connected to a motor mounted on the test tube holder. However, the problem in this patent is that the crank slider mechanism or eccentric wheel mechanism will shake or vibrate, or even swing significantly during transport even if the motor is not started, which will cause failed fixing of the test tube stand and damage to the test tube stand due to collision with other adjacent components. As a result, it is probably that the test tube stand cannot be further used or its life is greatly reduced even if it can be used. Secondly, the motor needs to control not only the transmission device that drives the test tube stand to move up and down, but also the drive device that drives the test tube holder to move horizontally, so that it is relatively complicated to control the motion of the test tube stand. Furthermore, the motor may start in the shaking or collision process of the PCR instrument during transport, thus causing operation of the transmission device and the drive device, however, there's no remedial action to stop the operation of the motor.

Chinese patent CN105039155B discloses that a drive nut drives a hot cover bracket fixed thereon to move up and down, so as to drive a hot cover to move up and down; when the hot cover moves down, it presses down the test tube stand, which in turn drives a connecting bracket to press down a spring, so that the sample on the test tube stand enters a detection module for analysis, and after the end of the analysis, the connecting bracket automatically rebounds under the drive of the spring, to drive the test tube stand to disengage from the detection module. This patent also has the problem of shaking or vibration of assemblies such as the test tube stand and the hot cover bracket during transport.

Therefore, it is necessary to provide a solution to reduce damage to instrument apparatuses during transport.

### Summary of the Invention

A first objective of the present invention is to provide an internal fixing device for an apparatus in a transport mode, wherein the internal fixing device comprises a motor, a transmission device, a limiting device and a microswitch; the motor comprises a motor output shaft, the transmission device comprises an eccentric roller, a roller output shaft, a rocking handle, a shoulder screw and a guide rail, the motor output shaft is fixedly connected to the eccentric roller, the eccentric roller is movably connected to the rocking handle through the roller output shaft, the rocking handle is movably connected to the guide rail through the shoulder screw, and the rocking handle can move up and down relative to the guide rail and swing clockwise and counterclockwise relative to the shoulder screw; the limiting device is disposed near the end of the rocking handle, and when the rocking handle is in a vertical state, the limiting device has an appropriate distance from this end of the rocking handle; and said apparatus comprises a transport mode, and when the apparatus is in the transport mode, the rocking handle triggers the microswitch, the motor stops operation, and the limiting device prevents the motion of the rocking handle.

As a further improvement, the internal fixing device comprises two transmission devices disposed on two sides of the apparatus respectively, the two ends of the motor output shaft being fixed to the eccentric rollers of the two transmission devices respectively.

As a further improvement, the internal fixing device further comprises a sliding chute movably connected to the shoulder screw, the sliding chute being connected to said guide rail in a sliding manner.

As a further improvement, the internal fixing device is provided with at least two optocoupler switches near the eccentric roller respectively, for controlling the on and off of the motor.

As a further improvement, the motor is a DC motor.

A second objective of the present invention is: to provide a detection analyzer, comprising a support frame and a motion system fixed to the support frame, wherein the motion system comprises a motor, a motor output shaft connected to the motor, an eccentric roller fixedly connected to the motor output shaft, a rocking handle movably connected to the eccentric roller, and a guide rail movably connected to the rocking handle; the rocking handle can move up and down relative to the guide rail and can also move clockwise and counterclockwise relative to its connection point with the guide rail; a limiting device and a microswitch are disposed near the end of the rocking handle; when the rocking handle is in a vertical state, said end of the rocking handle is spaced from the limiting device at an appropriate distance; and when the rocking handle triggers the microswitch, the motor stops operation and the limiting device rests against said end of the rocking handle.

As a further improvement, said motion system comprises two eccentric wheels fixedly connected to the two ends of the motor output shaft respectively, two rocking handles, and two guide rails, each eccentric wheel being movably connected to the corresponding rocking handle and guide rail in turn.

As a further improvement, the connection point of the rocking handle and the guide rail is located in the middle of the rocking handle, and the rocking handle is movably connected to the guide rail via a sliding chute.

As a further improvement, said detection analyzer is a nucleic acid detection analyzer.

As a further improvement, further comprised is a position identification system, which comprises a position identification device for determining that a sample compartment is in a standby mode, a position identification device for determining that the sample compartment is in a working mode, and a position identification device for determining that the sample compartment is in a transport mode.

As a further improvement, said motor is a DC motor.

A third objective of the present invention is to provide a detection and analysis apparatus comprising a support frame and a motion system fixed to the support frame, wherein the motion system comprises a motor, a motor output shaft connected to the motor, an eccentric roller fixedly connected to the motor output shaft, a rocking handle movably connected to the eccentric roller, and a guide rail movably connected to the rocking handle; said analyzer comprises at least three modes: a standby mode, a working mode, and a transport mode, wherein when the analyzer is in the transport mode, the motor, the motor output shaft, the eccentric roller, the rocking handle and the guide rail form self locking and cannot move relative to each other.

As a further improvement, the rocking handle can move up and down relative to the guide rail, and can swing clockwise and counterclockwise relative to its connection point with the guide rail.

As a further improvement, a limiting device and a microswitch are disposed near the end of the rocking handle; when the rocking handle is in a vertical state, said end of the rocking handle is spaced from the limiting device at an appropriate distance; and when the rocking handle triggers the microswitch, the motor stops operation and the limiting device rests against said end of the rocking handle.

As a further improvement, said motion system comprises two eccentric wheels fixedly connected to the two ends of the motor output shaft respectively, two rocking handles, and two guide rails, each eccentric wheel being movably connected to the corresponding rocking handle and guide rail in turn.

As a further improvement, the connection point of the rocking handle and the guide rail is located in the middle of the rocking handle, and the rocking handle is movably connected to the guide rail via a sliding chute.

As a further improvement, the motor is a DC motor.

A fourth objective of the present invention is to provide a method for reducing damage to an apparatus during transport, comprising: providing an apparatus, wherein the apparatus comprises a support frame and a motion system fixed to the support frame; the motion system comprises a motor, a motor output shaft connected to the motor, an eccentric roller fixedly connected to the motor output shaft, a rocking handle movably connected to the eccentric roller, and a guide rail movably connected to the rocking handle; a limiting device and a microswitch are disposed near the end of the rocking handle, and when the rocking handle is in a vertical state, the limiting device has an appropriate distance from the end of the rocking handle; the apparatus comprises a transport mode; and when the device is set into the transport mode, the rocking handle triggers the microswitch, the motor stops operation, the limiting device prevents the motion of the rocking handle, and the motor, the motor output shaft, the eccentric roller, the rocking handle, and the guide rail form self locking and cannot move relative to each other.

As a further improvement, the apparatus further comprises a working mode, and when the apparatus is set into the working mode, the rocking handle disengages from the microswitch, the apparatus releases the self locking, and the rocking handle moves up and down relative to the guide rail, and also swings clockwise and counterclockwise relative to its connection point with the guide rail. As a further improvement, said motion system comprises two eccentric wheels fixedly connected to the two ends of the motor output shaft respectively, two rocking handles, and two guide rails, each eccentric wheel being movably connected to the corresponding rocking handle and guide rail in turn.

As a further improvement, the connection point of the rocking handle and the guide rail is located in the middle of the rocking handle, and the rocking handle is movably connected to the guide rail via a sliding chute.

As a further improvement, the motor is a DC motor.

The nucleic acid detection analyzer described in the present invention can be provided with a standby mode, a working mode and a transport mode. When the instrument is in the standby mode, the sample compartment is in the lowest position, and can be pulled out manually (or electronically). The sample compartment is provided therein with a porous tube holder in which test tubes containing test reagents can be placed. The rocking handle in an eccentric roller assembly is disengaged from contact with the microswitch mounted on a limiting block. When the instrument is in a working experiment state, the sample compartment ascends to the highest position when the working experiment is started, and forms a PCR amplification system together with a hot cover mounted on the base of a transmission platform of the instrument, so as to complete the temperature control process for a predetermined temperature curve. The rocking handle in an eccentric roller assembly is disengaged from contact with the microswitch mounted on a limiting block. When the instrument is in the transport mode, in order to prevent shaking of the sample compartment and ensure reliability of transport, it is required to fix the sample compartment. When the instrument starts the transport mode, 4 rows of 8-linked tubes are put when the sample compartment is at the lowest position, the DC motor rotates and causes the sample compartment to ascend vertically to the highest working position through motion conversion, and at this point, a spring inside the sample compartment is compressed and the spring pressure brings the sample compartment into close contact with the base of the transmission platform. The motor continues to move and the rocking handle in the right eccentric roller assembly continues to swing until the microswitch on the limiting block is triggered to output a signal to stop working of the DC motor, and at this point, the sample compartment reaches a transport position and the instrument is in the transport mode. The spring inside the sample compartment is still in the compressed state, and provides a sufficient spring pressure to ensure that the sample compartment is in a stable fixed state.

When the instrument is subjected to external vibration or impact in the vertical direction during transport, the sample compartment will not cross the upper dead center of the transmission mechanism under the combined action of an internal spring pressure and a downward impact force to cause reverse rotation of the motor, so that the sample compartment is in an unfixed state; and meanwhile, the limiting device further prevents further forward rotation of the motor resulted from exogenic action, so that the sample compartment is always in a stable fixed state.

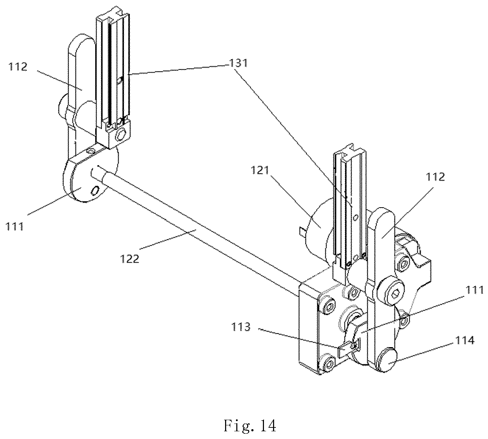

The present invention has the following beneficial effects: the motion principle of the present invention is that the motor drives the two eccentric roller assemblies through the motor output shaft to convert the circular motion of the motor into the left and right swing of the rocking handles in the left and right eccentric roller assemblies, while the guide rails slide up and down in the sliding chutes. The guide rails are located on the two sides of the sample compartment of the apparatus, and with the up-and-down reciprocating motion of the guide rails, the sample compartment is in the standby mode, working mode or transport mode, as shown in Fig. 14.

With interworking of the eccentric roller assemblies, the motor assembly, the microswitch and the limiting block, the above members form self locking together under the action of external vibration or impact in the vertical direction during transport, and the members cannot move relative to each other, thus avoiding reverse rotation or continuous rotation of the motor so that the sample compartment is in an unfixed state to generate displacement. Therefore, it is ensured that the sample compartment is always in a fixed position during transport of the fluorescent quantitative PCR instrument, which reduces the probability and extent of damage to the instrument during transport and in turn well protects the instrument.

### Brief Description of the Drawings

Fig. 1 is a schematic diagram of a standby mode.
Fig. 2 is a front view of the interior in a standby mode.
Fig. 3 is a side view of the rear of the interior in a standby mode.
Fig. 4 is a side view of the interior in a standby mode.
Fig. 5 is a schematic diagram of a working mode.
Fig. 6 is a front view of the interior in a working mode.
Fig. 7 is a side view of the rear of interior in a working mode.
Fig. 8 is a side view of the interior in a working mode.
Fig. 9 is a schematic diagram of a transport mode.
Fig. 10 is a front view of the interior in a transport mode.
Fig. 11 is a side view of the rear of interior in a transport mode.
Fig. 12 is a side view of the interior in a transport mode.
Fig. 13 is a structure diagram of the interior of a nucleic acid detection analyzer.
Fig. 14 is a schematic diagram of the motion.

### Detailed Description of the Embodiments

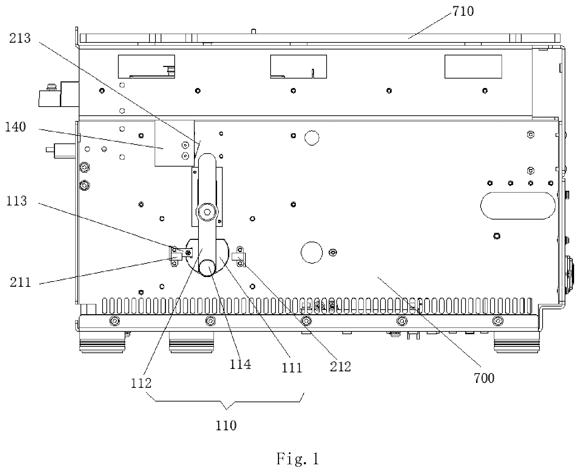

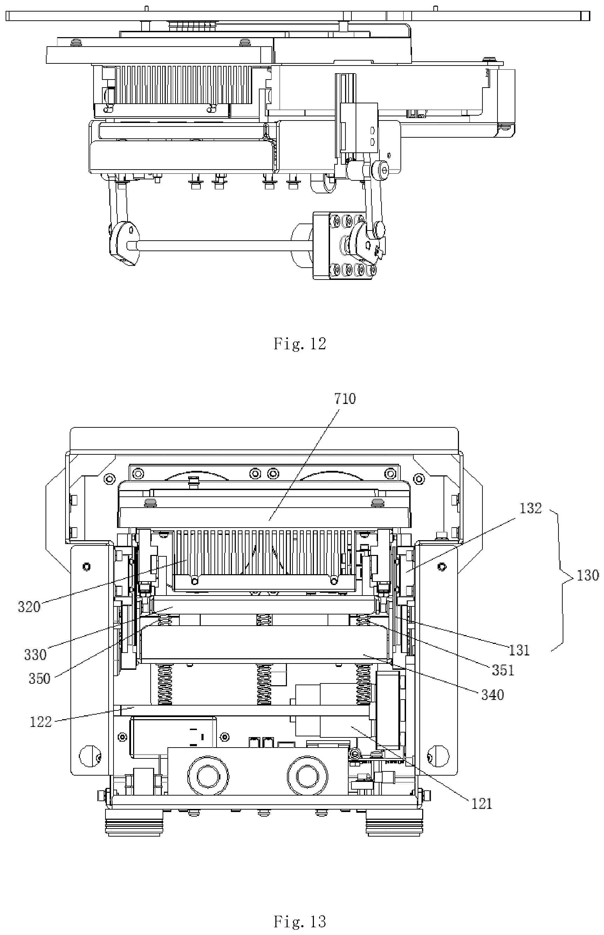

An internal fixing device for an apparatus in a transport mode, as shown in Figs. 1 and 14, includes a motor 121, transmission devices 110, a limiting device 140 and a microswitch 213. The two transmission devices 110 are disposed on the two sides of the apparatus respectively, and the transmission devices 110 each include an eccentric roller 111, a rocking handle 112 and a guide rail 131. The motor 121 includes a motor output shaft 122, the two ends of which are fixedly connected to the left and right eccentric rollers 111 respectively; each eccentric roller 111 is movably connected to the corresponding rocking handle 112 through a roller output shaft 114; and the rocking handle 112 is movably connected to the corresponding guide rail 131 through a shoulder screw 115. The fixed connection described in this patent means that the interconnected members cannot displace or rotate relative to each other, but can only displace or rotate relatively as a whole. The movable connection described in this patent means that the interconnected members not only can displace or rotate relatively as a whole, but also can displace or rotate relative to each other. The limiting device 140 is disposed near the end of one of the rocking handles 112, and when the transmission device 110 is at the upper dead center, the rocking handle 112 is in a vertical state, and the end of the rocking handle 112 has an appropriate distance from the limiting device 140. This distance ensures that the rocking handle can swing to the limiting device from the upper dead center, and can prevent further forward rotation of the motor resulted from exogenic action through the limiting device, so that the apparatus is in a stable self-locking state. In one embodiment, the microswitch 213 is disposed near the limiting device and the end of the rocking handle 112, or can be directly disposed on the limiting device 140. When this rocking handle 112 reaches the upper dead center and continues to swing until the microswitch 213 is triggered, the motor 121 stops rotating. The microswitch 213 may also not be mounted on the limiting device 140, but is disposed between the limiting device 140 and the rocking handle 112. Said device for fixing the instrument further includes sliding chutes 132 that are in sliding fit with the guide rails 131 (as shown in Fig. 13).

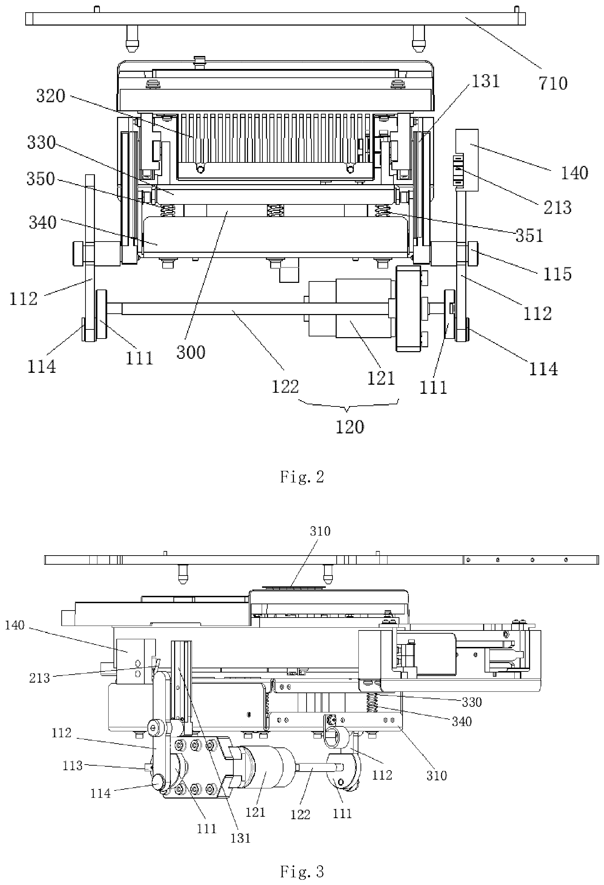

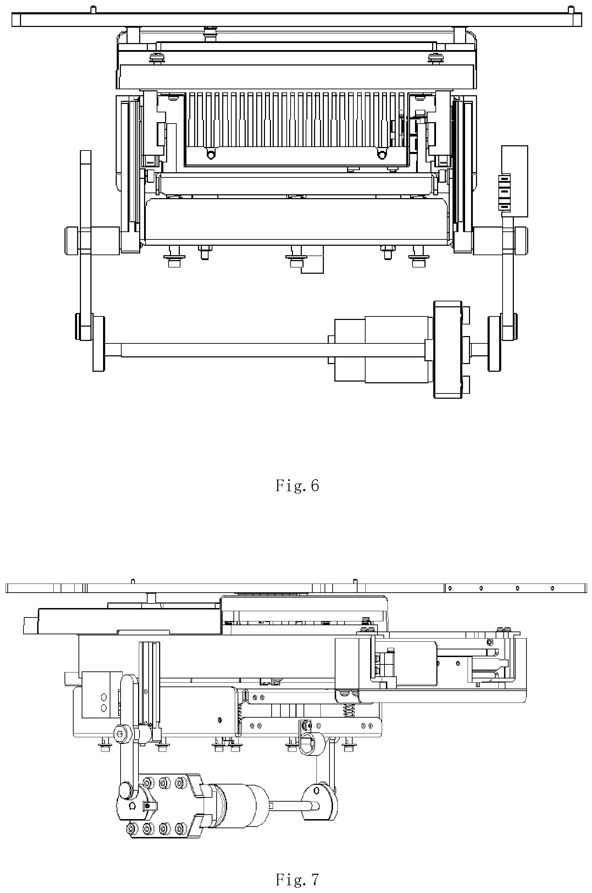

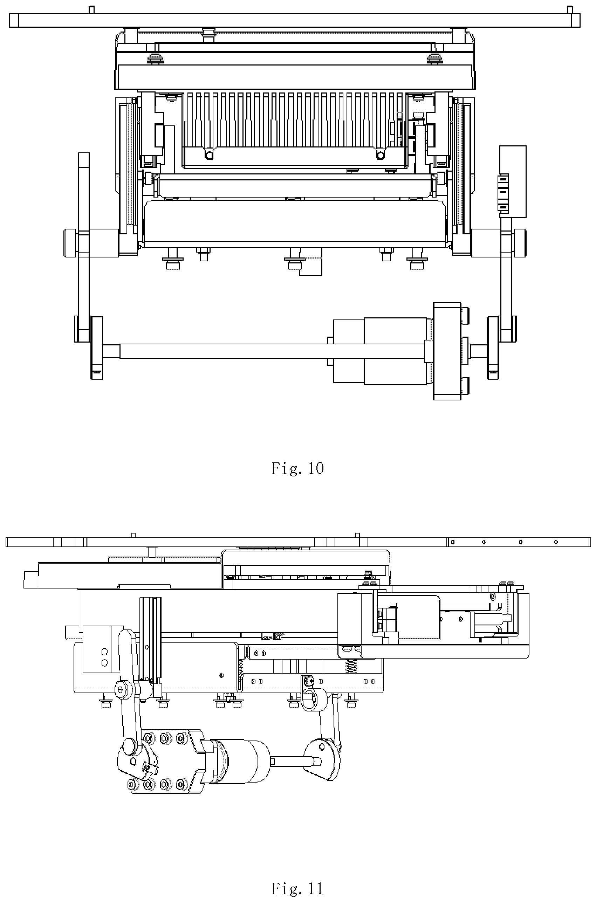

The nucleic acid detection analyzer, as shown in Figs. 1 to 13, includes a motion system, a sample compartment 300, an optical system, and a support frame 700.

The sample compartment 300 includes a sample block (not shown in the figures) for storage of test tubes 310, a thermal circulation device, a fan mounting plate 330, and a bottom plate 340 that is connected to the guide rails 131.

An energy storage device, selected from but not limited to a spring 350, is disposed between the fan mounting plate 330 and the bottom plate 340. A spring guide post 351 is installed at the bottom of the fan mounting plate 330, and is sleeved in the spring 350. The bottom plate 340 is provided with a hole for the spring guide post 351 to pass through. When the bottom plate 340 moves upward, the bottom plate 340 pushes the spring 350 placed thereon to move upward, and then the spring 350 pushes the fan mounting plate 330 disposed thereon to move upward, until the sample compartment 300 ascends to the highest position and the test tube 310 and a support platform 710 rest against each other. At this point, the motor 121 stops rotating and the nucleic acid detection analyzer enters the working mode to complete the temperature control process for a predetermined temperature curve and the detection of a PCR reaction process. In one solution, a hot cover is disposed above the sample compartment. The thermal circulation device includes a heat sink 320 and a Peltier (not shown).

The two transmission devices are disposed on the two sides of the sample compartment 300 respectively, and each transmission device includes an eccentric roller 111, a rocking handle 112 and a guide rail 131. The two ends of the motor output shaft 122 are fixed to the eccentric rollers 111 on the left and right sides respectively, the eccentric rollers 111 are movably connected to the rocking handles 112 through the roller output shafts 114, and the guide rails 131 are movably connected to the rocking handles 112 by the shoulder screws 115 and are in sliding fit with the sliding chutes 132. The limiting device 140 is mounted on the right of a support frame 700, and is located on the left of the rocking handle 112 when the transmission device is at the upper dead center. An appropriate distance is set between the limiting device 140 and the rocking handle 112 in this state, and this distance ensures that after the rocking handle 112 swings from the upper dead center to the limiting device 140, the limiting device 140 can prevent further forward rotation of the motor 121 resulted from exogenic action, so that the sample compartment 300 is in a stable self-locking state in the transport mode. The microswitch 213 is disposed on the limiting device 140; and when the rocking handle 112 reaches the upper dead center and continues to swing to trigger the microswitch 213, the motor 121 stops rotating.

The motor 121 of the present invention is preferably a DC motor. The two ends of the motor output shaft 122 are fixedly connected to the eccentric rollers 111 on the two sides of the apparatus to convert the circular periodic motion of the motor 121 into the upward and downward linear motion of the sample compartment 300.

The motor 121, the fan mounting plate 330, the bottom plate 340, the spring 350 and the support platform 710 jointly form a force application device of the analyzer for upper and lower transmission.

Depending on the use condition of the nucleic acid detection analyzer, the analyzer is in different states such as a standby state, a working state for running detection, and a transport mode. In different states of the analyzer, the sample compartment is located in different positions in the analyzer. When the analyzer is in the standby state, the sample compartment is in its lower position in the analyzer. When the analyzer is in the working state, the sample compartment is in its higher position in the analyzer. When the nucleic acid detection analyzer is carried, the sample compartment is in a position in the analyzer slightly lower than that in the working mode, but in a position higher than that in the standby state.

In order to determine the state of the analyzer, the nucleic acid detection analyzer is further provided with a position identification system 200 to determine the position of the sample compartment in the analyzer. Said identification system includes a first optocoupler switch 211 to determine that the sample compartment is in the standby mode, a second optocoupler switch 212 to determine that the sample compartment is in the working mode, and a microswitch 213 to determine that the sample compartment is in the transport mode. Said first optocoupler switch 211 and second optocoupler switch 212 are mounted on the support frame and located on the left and right sides of the eccentric roller, respectively. After the eccentric roller rotates to drive the rocking handle to ascend to the highest position, the eccentric roller continues to rotate in the same direction, the microswitch 213 is disposed in the direction that the rocking handle continues to swing, and the microswitch deviates from the vertical position of the rocking handle. The position identification device includes, but is not limited to, an optocoupler switch, a microswitch, a position sensing switch, etc.

As shown in Figs. 1 to 4, when the analyzer selects the standby mode, the rocking handle and the roller output shaft 114 of the eccentric roller are located in the lowest position, and at this point, a stop bar 113 exactly blocks the optical path of the first optocoupler switch 211. The control system of the analyzer obtains a signal that the optical path of the first optocoupler switch is blocked up, the motor will not rotate, and the detection system also will not operate.

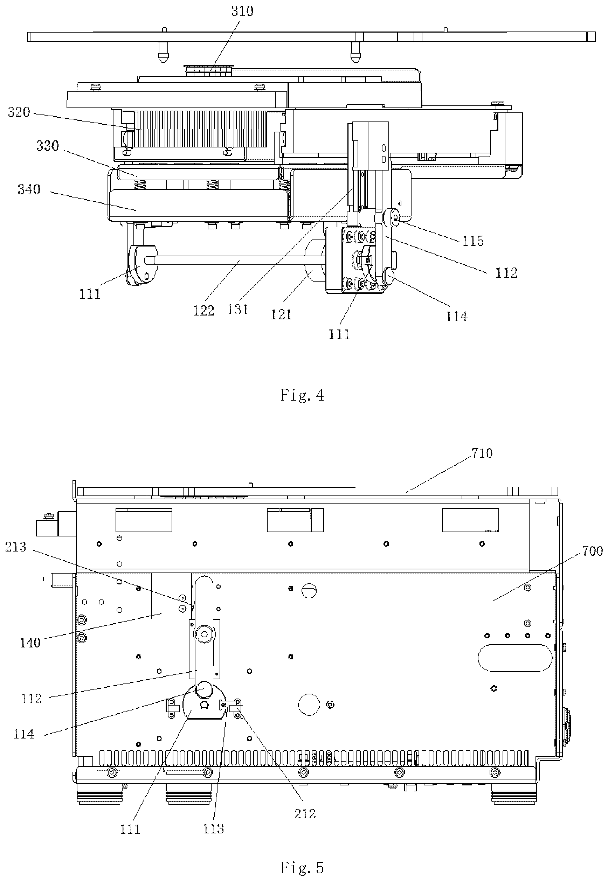

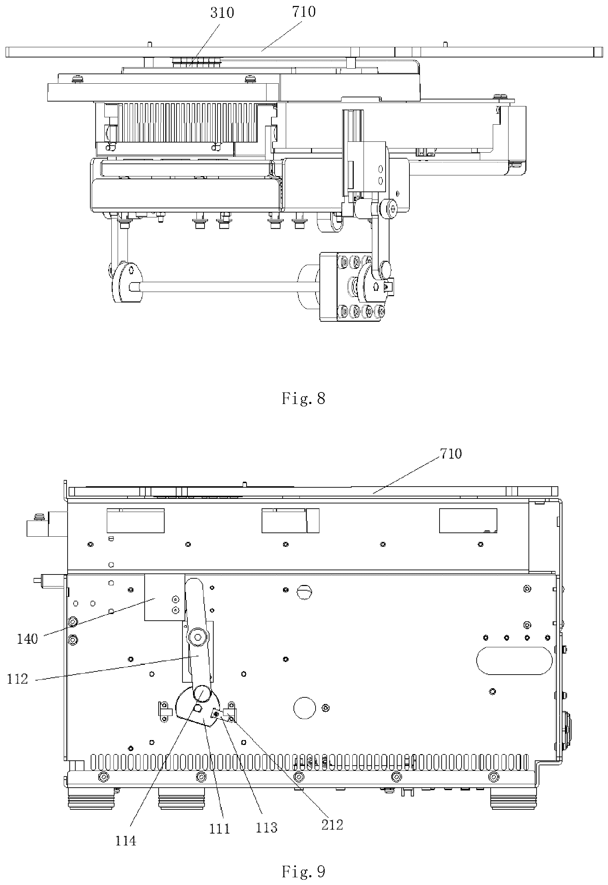

As shown in Figs. 5 to 8, when the analyzer selects the working mode, the motor 121 is started to run through the control system of the analyzer to realize forward rotation of the motor, and the circular motion of the motor drives clockwise rotation of the eccentric roller 111 and swing of the rocking handle 112. The guide rail 131 moves upward in the sliding chute 132 along with the swing of the rocking handle. The bottom plate 340 moves upward synchronously with the guide rail. The bottom plate 340 pushes the spring 350 mounted thereon upward, the spring in turn pushes the fan mounting plate 330 in contact therewith upward to the highest position of the sample compartment, and the test tubes placed in a sample block press against the support platform, which applies a downward pressure to the sample compartment. The sample compartment is stably kept in a working position via the downward pressure applied by the support platform to the sample compartment and the spring pressure. Specifically, the eccentric roller rotates 90 degrees clockwise from the standby mode, and the upper section of the rocking handle swings to the right at first. The eccentric roller further rotates 90 degrees clockwise, and the upper section of the rocking handle swings to the left. When the transmission device is at the upper dead center, the stop bar 113 exactly blocks the second optocoupler switch 212. The control system of the analyzer obtains information that the optical path of the second optocoupler switch is blocked up, the motor stops running, and the detection is started. In order to prevent shaking of the sample compartment and ensure reliability of transport, it is required to fix the sample compartment during transport. As shown in Figs. 9 to 12, when the analyzer selects the transport mode, the control system first leaves the sample compartment in the position in the standby mode, and an appropriate number of empty test tubes 310 (e.g., 4 rows of 8-linked tubes) are placed in the sample compartment. Then the motor is started to rotate, and as the motor rotates, the sample compartment ascends vertically to the position in the working state. At this point, the spring 350 between the bottom plate and the fan mounting plate is compressed. The motor continues the forward rotation, and the rocking handle passes the upper dead center, and then swings and touches the microswitch 213 to trigger the microswitch to work. After the control system obtains the signal that the microswitch is triggered, the motor stops rotating. Since the rocking handle does not deviate much from the upper dead center, the spring between the bottom plate and the fan mounting plate is still in the compressed state, and there is enough spring pressure to ensure that the sample compartment is in a stable fixed position.

When the analyzer is in the transport mode, the motor stops rotating, and the eccentric roller cannot continue to rotate clockwise or counterclockwise with the help of rotation of the motor. When the apparatus has an accident during transport and is subjected to external vibration or impact in the vertical direction, the action line of force, in the vertical direction, of the eccentric roller assembly of the instrument apparatus fixed by this device will not deviate from the position of the upper dead center, thus avoiding generating the torque under the combined action of the internal spring pressure and the downward impact force to cause reverse rotation or further rotation of the motor, which ensures that the instrument apparatus is always in a fixed position during transport and avoids the risk of damage to the instrument in transport.

When the apparatus is subjected to external vibration or impact in the vertical direction during transport, it may cause the rocking handle to accidentally further swing to the left from the position in the transport mode to the position where the internal spring loses pressure, so that the sample compartment is in an unfixed state to generate displacement. However, such unexpected swing of the rocking handle will not occur due to the restriction of the limiting device. In addition, in the case of no lifting force, the rocking handle will not reverse to the right to reach the highest position and continue to reverse to the position where the internal spring loses pressure. Therefore, it ensures that the sample compartment is in a stable fixed position. The side of the limiting device, on which the rocking handle is disposed, can be adjusted according to the actual design.

The control system of the nucleic acid detection analyzer changes the positive and negative poles of the DC motor, so that the motor rotates reversely, the circular motion of the motor drives the eccentric roller 111 to rotate counterclockwise, and the rocking handle 112 connected with the eccentric roller swings. The guide rail 131 moves downward in the sliding chute 132 along with the swing of the rocking handle. The bottom plate is driven by the guide rail to move downward synchronously. The spring mounted on the bottom plate moves downward, and the test tube holder moves downward synchronously until the sample compartment returns to the position in the standby state. Specifically, the eccentric roller rotates 90 degrees counterclockwise from the position in the working mode or in the transport mode, and the upper section of the rocking handle swings to the right. The eccentric roller further rotates 90 degrees clockwise, and the upper section of the rocking handle swings to the left, until the stop bar 113 blocks the optical path of the first optocoupler switch 211 again. The control system of the analyzer obtains data that the optical path of the first optocoupler switch is blocked up, determines that the sample compartment has returned to the position in the standby mode, and stops rotation of the motor.

The optical system of the nucleic acid analyzer includes a light source and a detector (e.g., a photomultiplier tube). The excitation light generated by the light source excites fluorescent dyes or fluorophore-containing probes in the test tube to produce fluorescence, and the resulting fluorescence can be detected by the detector. The thermal cycling module of the analyzer heats and cools the test tube holder, thus the reaction reagent in the test tube circulates among denaturation, annealing and extension temperatures.

The directional words such as "upper", "lower", "left", "right", "clockwise" and "counterclockwise" that are involved in the present invention are relative positions of various components, rather than absolute spatial positions and limit to the present invention.

## Claims

1. An internal fixing device for an apparatus in a transport mode, wherein the internal fixing device comprises a motor, a transmission device, a limiting device and a microswitch; the motor comprises a motor output shaft, the transmission device comprises an eccentric roller, a roller output shaft, a rocking handle, a shoulder screw and a guide rail, the motor output shaft is fixedly connected to the eccentric roller, the eccentric roller is movably connected to the rocking handle through the roller output shaft, the rocking handle is movably connected to the guide rail through the shoulder screw, and the rocking handle can move up and down relative to the guide rail and swing clockwise and counterclockwise relative to the shoulder screw; the limiting device is disposed near the end of the rocking handle, and when the rocking handle is in a vertical state, the limiting device has an appropriate distance from this end of the rocking handle; and said apparatus comprises a transport mode, and when the apparatus is in the transport mode, the rocking handle triggers the microswitch, the motor stops operation, and the limiting device prevents the motion of the rocking handle.

2. The internal fixing device according to claim 1, comprising two transmission devices disposed on two sides of the apparatus respectively, the two ends of the motor output shaft being fixed to the eccentric rollers of the two transmission devices respectively.

3. The internal fixing device according to claim 2, further comprising a sliding chute movably connected to the shoulder screw, the sliding chute being connected to said guide rail in a sliding manner.

4. The internal fixing device according to claim 3, wherein at least two optocoupler switches are disposed near the eccentric roller respectively, for controlling the on and off of the motor.

5. A detection analyzer, comprising a support frame and a motion system fixed to the support frame, wherein the motion system comprises a motor, a motor output shaft connected to the motor, an eccentric roller fixedly connected to the motor output shaft, a rocking handle movably connected to the eccentric roller, and a guide rail movably connected to the rocking handle; the rocking handle can move up and down relative to the guide rail and can also swing clockwise and counterclockwise relative to its connection point with the guide rail; a limiting device and microswitch are disposed near the end of the rocking handle; when the rocking handle is in a vertical state, said end of the rocking handle is spaced from the limiting device at an appropriate distance; and when the rocking handle triggers the microswitch, the motor stops operation and the limiting device rests against said end of the rocking handle.

6. The detection analyzer according to claim 5, wherein said motion system comprises two eccentric rollers fixedly connected to the two ends of the motor output shaft respectively, two rocking handles, and two guide rails, each eccentric roller being movably connected to the corresponding rocking handle and guide rail in turn.

7. The detection analyzer according to claim 6, wherein the connection point of the rocking handle and the guide rail is located in the middle of the rocking handle, and the rocking handle is movably connected to the guide rail via a sliding chute.

8. The detection analyzer according to claim 7, wherein said detection analyzer is a nucleic acid detection analyzer.

9. A detection and analysis apparatus, comprising a support frame and a motion system fixed to the support frame, wherein said motion system comprises a motor, a motor output shaft connected to the motor, an eccentric roller fixedly connected to the motor output shaft, a rocking handle movably connected to the eccentric roller, and a guide rail movably connected to the rocking handle; said analyzer comprises at least three modes: a standby mode, a working mode, and a transport mode, wherein when the analyzer is in the transport mode, the motor, the motor output shaft, the eccentric roller, the rocking handle and the guide rail form self locking and cannot move relative to each other.

10. The detection and analysis apparatus according to claim 9, wherein the rocking handle can move up and down relative to the guide rail, and can swing clockwise and counterclockwise relative to its connection point with the guide rail.

11. The detection and analysis apparatus according to claim 10, wherein a limiting device and a microswitch are disposed near the end of the rocking handle; when the rocking handle is in a vertical state, said end of the rocking handle is spaced from the limiting device at an appropriate distance; and when the rocking handle triggers the microswitch, the motor stops operation and the limiting device rests against said end of the rocking handle.

12. The detection and analysis apparatus according to claim 11, wherein said motion system comprises two eccentric rollers fixedly connected to the two ends of the motor output shaft respectively, two rocking handles, and two guide rails, each eccentric roller being movably connected to the corresponding rocking handle and guide rail in turn.

13. The detection and analysis apparatus according to claim 12, wherein the connection point of the rocking handle and the guide rail is located in the middle of the rocking handle, and the rocking handle is movably connected to the guide rail via a sliding chute.

14. The detection and analysis apparatus according to claim 11, further comprising a sample compartment and a sample compartment position identification system, which comprises a position identification device for determining that a sample compartment is in a standby mode, a position identification device for determining that the sample compartment is in a working mode, and a position identification device for determining that the sample compartment is in a transport mode.

15. The detection and analysis apparatus according to claim 11, wherein said motor is a DC motor.

16. A method for reducing damage to an apparatus during transport, wherein an apparatus is provided, comprising a support frame and a motion system fixed to the support frame; said motion system comprises a motor, a motor output shaft connected to the motor, an eccentric roller fixedly connected to the motor output shaft, a rocking handle movably connected to the eccentric roller, and a guide rail movably connected to the rocking handle; a limiting device and a microswitch are disposed near the end of the rocking handle, and when the rocking handle is in a vertical state, the limiting device has an appropriate distance from the end of the rocking handle; said apparatus comprises a transport mode; and when the device is set into the transport mode, the rocking handle triggers the microswitch, the motor stops operation, the limiting device prevents the motion of the rocking handle, and the motor, the motor output shaft, the eccentric roller, the rocking handle, and the guide rail form self locking and cannot move relative to each other.

17. The method according claim 16, further comprising a working mode, wherein when the apparatus is set into the working mode, the rocking handle disengages from the microswitch, the apparatus releases the self locking, and the rocking handle moves up and down relative to the guide rail, and also swings clockwise and counterclockwise relative to its connection point with the guide rail.

18. The method according to claim 17, wherein said motion system comprises two eccentric rollers fixedly connected to the two ends of the motor output shaft respectively, two rocking handles, and two guide rails, each eccentric roller being movably connected to the corresponding rocking handle and guide rail in turn.

19. The method according to claim 18, wherein the connection point of the rocking handle and the guide rail is located in the middle of the rocking handle, and the rocking handle is movably connected to the guide rail via a sliding chute.
